(19) Europäisches Patentamt
European Patent Office
Office européen des brevets

(11) EP 1 068 889 A1

(12) **EUROPEAN PATENT APPLICATION**

(43) Date of publication:
**17.01.2001 Bulletin 2001/03**

(51) Int Cl.[7]: **B01D 39/16**, A61M 16/10

(21) Application number: **99202349.9**

(22) Date of filing: **16.07.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **3M Innovative Properties Company**
**St. Paul, Minnesota 55133-3427 (US)**

(72) Inventors:
- **De Jong, Guido**
**4815 HK Breda (NL)**
- **Krapp, Jan Thomas**
**4815 HK Breda (NL)**

(74) Representative: **Voortmans, Gilbert J.L. et al**
**3M Europe S.A.,**
**Hermeslaan 7**
**1831 Diegem (BE)**

(54) **High efficiency medical breathing system filter based on a filtration medium of a nonwoven web of thermoplastic resin fibers**

(57) The present invention provides a filter device for connection to the respiratory tract of a patient, comprising a housing having a fluid chamber connecting to two fluid ports, the fluid chamber having a volume of not more than 140 ml and comprising a pleated filtration medium for capturing bacteria and viral material. The filter device is characterised in that the pleated filtration medium comprises a nonwoven web of electret charged fibers of a nonconductive thermoplastic resin, having a resistivity of at least $10^{14}$ ohm-cm, the electret charged fibers having an effective diameter (EFD) of not more than 5 µm and the filter device having an efficiency of at least 99.97% for sodium chloride particles of 0.3µm at a flow challenge of 30 l/min.

The filter device of the present invention can replace a glass fiber based filter commonly used in medical procedures offering similar or improved efficiency in removing microorganisms from the air at a lower pressure drop. The filter device of the present invention can further be provided in various shapes including a substantially cylindrical shape and the dead volume of the filter device can be reduced.

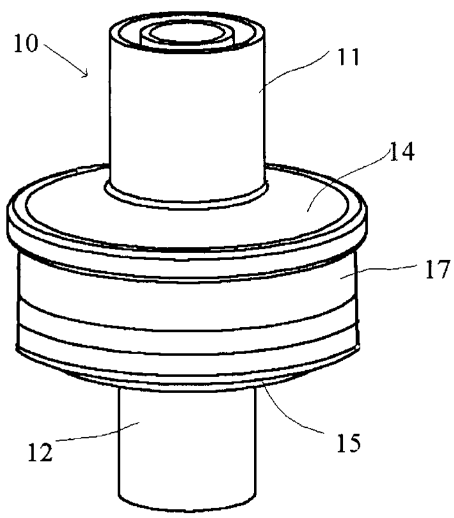


Figure 1

EP 1 068 889 A1

## Description

1. Field of the invention

The present invention relates to a filter device that is suitable for use as a high efficiency medical breathing system filter, in particular a filter device that can be used in combination with a breathing apparatus. The filter device of the invention comprises a pleated filtration medium of thermoplastic resin fibers and avoids the need for the use of a glass fiber based filtration medium.

2. Background of the invention.

In certain medical procedures such as intubation or tracheostomy the upper airways of a patient are bypassed. To avoid bacterial or virus infection of the patient in such a procedure, the inspired air from a ventilating apparatus or breathing apparatus needs to be filtered. Furthermore, the expired air leaving the patient needs to be filtered as well to avoid contamination of the breathing apparatus which may cause cross-contamination of patients.

Typically, the art has been using high efficiency filter devices to provide the necessary filtration. Often, such filter devices are also combined with a heat and moisture exchange filter that is intended to simulate the heat and moisture exchange that takes place during normal nasal breathing. Such filter devices are disclosed in for example WO 99/03525, GB 2.267.661, WO 94/02192 and US 5.320.096. The high efficiency filter devices employed typically comprise a housing having a fluid chamber with two fluid ports which are generally at opposite sides of the chambers. The fluid chambers of the prior art filter devices in these medical applications generally contain a pleated filtration medium that is based on glass fibers or ceramic fibers.

Glass fiber based filters have indeed been found to be highly effective in removing bacteria and viral material from the inspired and expired air. They furthermore have a high hydrostatic pressure which is desirable to avoid that when the patient vomits or ejects blood, such fluids do not readily pass the filter which could cause contamination of the breathing apparatus.

On the other hand, the glass fiber based filters also provide several disadvantages. For example, a large pressure drop is associated with these filters. Further, the filter is extensively pleated with the result that a fairly large fluid chamber is needed. Accordingly, such filters generally have a large dead volume, i.e. an amount of air will remain within the chamber without frequent refreshing. Additionally, it is difficult to produce the pleated glass fiber filtration medium in a circular form which would allow it to be used in housings that have a cylindrically shaped chamber. Accordingly, the glass fiber based filter devices are generally provided as a box like housing which is uncomfortable for the patient. An example of such a box like filter device for medical purposes is for example disclosed in US 3,815,754. Finally, when the glass fiber medium is pleated, cut and put into a housing, the glass fibers may shear or splinter and glass particles may thereby dislodge from the medium and become airborne.

Glass fiber based filters have also been used as the filtration medium for other applications such as respirators, face masks and in protecting certain devices such as computer hard disks against dust. Attempts have been made to replace glass fiber based filters in these applications. For example, US 4,824,451 discloses forming a high efficiency performance type filter having efficiencies ranging from 95% to 99.26% at pressure drops of about 8mm $H_20$, using meltblown microfiber webs having extremely small effective fiber diameters. Increased efficiencies were obtained by subsequently calendering the webs, such that efficiencies of up to 99.57% could be reached at pressure drops of from around 10mm to 20mm $H_20$. Although the filtration medium of this patent has lower pressure drops than the fiberglass filtration medium, the efficiency does not reach the performance level needed for a medical breathing system filter.

U.S. Pat. No. 5,240,479 describes laminating four or more layers of blown microfiber filtration medium each having an effective fiber diameter of approximately 3.2 microns, a basis weight of 30 $g/m^2$, and a thickness of .009 inches. Each particular layer is indicated as being 88% efficient (against 0.3 micron particles at 5.3cm/s). By placing four of these filtration media in successive layers, filtration efficiencies comparable to glass fiber based media is alleged to be obtained. This filter is generally quite thick and difficult to pleat into a high density pleated structure.

US 4,650,506 describes extremely fine solution blown fiber medium can be quite thin and have a high filtration performance comparable to that of a glass fiber based filtration media. As this media lacks sufficient strength it is not self supporting.

Although in certain filter applications, glass fiber based media have been replaced by other media, filter devices for medical purposes still generally use glass fiber based filters in instances where it is necessary to remove bacteria and virus material at very high levels. Such filters are in particular used in combination with a breathing apparatus. Because of the disadvantages elaborated above of glass fiber based filtration media, it would be desirable to replace this filtration media with other filtration media. Such other filtration media should however at least have a similar bacteria and virus removal efficiency as the existing filters, can preferably be easy manufactured in a reliable and cost effective way and can preferably be manufactured in various shapes including substantial circular allowing it to be used

in substantially cylindrical filter chambers. The filtration medium of the present invention preferably also allows minimizing the volume of the filter chamber, has a high hydrostatic pressure and a low pressure drop.

3. Summary of the invention

The present invention provides a filter device for connection to the respiratory tract of a patient, comprising a housing having a fluid chamber connecting to two fluid ports, the fluid chamber having a volume of not more than 140ml and comprising a pleated filtration medium for capturing bacteria and viral material characterised in that the pleated filtration medium comprises a nonwoven web of electret charged fibers of a nonconductive thermoplastic resin, having a resistivity of at least $10^{14}$ ohm-cm, said electret charged fibers having an effective diameter (EFD) of not more than 5 µm and said filter device having an efficiency of at least 99.97% for sodium chloride particles of 0.3µm at a flow challenge of 30 l/min.

It was found that a filter device having the stated minimum efficiency and that comprises a pleated filtration medium based on a nonwoven web of electret charged fibers of a nonconductive thermoplastic resin provides the bacteria and viral removal efficiency similar or better than the commonly used glass fiber based filter devices, at generally lower pressure drops then the glass fiber based filter devices. Typically, the filter device of the invention has a pressure drop of less than 3.0 cm $H_2O$, preferably less than 2.0 cm $H_2O$ and most preferably less than 1.5 cm $H_2O$ at a flow challenge of 60 l/min. The filter device of the present invention further allows minimizing the volume of the fluid chamber. Further, the filtration medium used in the filter device of the invention can be provided in various shapes allowing the fluid chamber to be substantially cylindrical. The filtration medium of the filter device of the invention also has a hydrostatic pressure comparable or better than the existing glass fiber based filter devices.

The present invention further provides a method of filtering breathable air comprising the steps of passing breathable air to be inspired by a patient through the filter device of the invention and passing air expired by the patient through the filter device of the invention.

4. Brief description of the drawings

The following drawings will be referred to hereinafter to illustrate the invention without however any intention to limit the invention thereto:

Figure 1 is a three dimensional view of a preferred embodiment of a filter device 10 according to the invention.

Figure 2 is a cross-sectional view of the preferred embodiment shown in Figure 1.

5. Detailed description of the invention.

The filter device of the present invention comprises a housing having a fluid chamber connecting to two fluid ports which are preferably arranged at opposite sides of the fluid chamber. A preferred embodiment of the filter device is shown in figures 1 and 2. As shown in these figures, the filter device 10 comprises a fluid chamber consisting of a cylindrical portion 17 and funnel transition regions 14 and 15 at opposite sides of the cylindrical portion 17 and connecting to an inlet port 11 and an outlet port 12. The funnel transition regions may have an angle of 90° to 5° from the housing flow axis. A short slightly tapered transition zone is generally preferred. However, a housing design without the transition zones can also be used. The volume of the fluid chamber as measured according to ISO 9360 should not be more than 140ml and is preferably less than 100ml.

The inlet and outlet ports 11 and 12 are of a smaller diameter than the central portion 17 which is cylindrical or substantially cylindrical in the preferred embodiment but which may also be of a different shape such as for example in a box like shape. The inlet and outlet ports are generally cylindrical but can be of different shape as well. When the filter device is in use, the inlet port 11 would be connected to a conduit such as a tube (not shown) by known means, which tube would then be in fluid communication with the trachea of a patient. At the opposite end, the outlet port 12 of the filter device would be connected to a second conduit such as a branched tube where two other ends of the branched tube would be connected to a breathing apparatus such as a ventilation device. In a ventilation device, inhalation air would pass down one leg of the branched tube then successively through the filter device, the first tube and into the patient. Exhalation air would successively pass through the first tube, the filter device and then through the other leg of the branched tube. This example of use of the filter device in combination with a particular breathing apparatus is however merely for illustration purposes as the filter device can also be used with other breathing apparatuses having other arrangements.

The cylindrical portion 17 of the filter device 10 comprises the pleated filtration medium 1. Preferably, the cylindrical portion 17 has a diameter between 30 and 90 mm. and a height between 5 and 30 mm. The pleated filtration medium 1 is sealed to the side walls of the fluid chamber 16. The required sealing may be accomplished by rotating the filter device including the filtration medium 1 and dropping an uncured sealant on a sealant dispersion member

(not shown) in the filter device 10. Centrifugal force will force the sealant radially outward toward the interface of the fluid chamber walls and the filtration medium 1. The sealant dispersion member helps guiding the uncured sealant to the proper location without wetting the filtration medium 1 unnecessarily. Subsequent or simultaneous curing of the sealant will result in the desired sealing.

The filter device 10 preferably comprises a housing in plastic. Accordingly, since the filtration medium 1 comprises a nonwoven web of thermoplastic resin fibers, it is also possible to seal the filtration medium 1 to the fluid chamber wall by ultrasonic welding such as described in US 5,512,172. As a further alternative, the filtration medium can be sealed to the fluid chamber wall while producing the housing by inject molding. After this insert molding process, the edges of the filtration medium will be embedded by the plastic material of the housing and a proper sealing is ensured. A suitable insert molding technique is described in US 5,679,122 and EP 713 421.

The filtration medium 1 is a charged nonwoven fiber web, which fibers are formed of a nonconductive thermoplastic resin having a resistivity of at least $10^{14}$ ohm-cm, preferably at least $10^{16}$ ohm-cm. Suitable nonconductive polymers include those that have the capability of possessing a non-transitory or long lived trapped charge. The polymer can be a homopolymer or copolymer or polymer blend. The preferred polymers include polyolefins; such as polypropylene, poly(4-methyl-1-pentene) or linear low density polyethylene; polystyrene; polycarbonate and polyester. The major component of the polymer or polymer blend is preferably polypropylene because of polypropylene's high resistivity, ability to form melt-blown fibers with diameters useful in this invention, satisfactory charge stability, hydrophobicity and resistance to humidity.

The fibers of the nonwoven web of the filtration medium 1 of the present invention are generally formed by melt blowing and preferably comprise a performance enhancing additive that increases the filtration performance of the filtration medium 1. Potential performance-enhancing additives include those described by Jones et al., U.S. Pat. No. 5,472,481 and Rousseau et al., WO 97/07272 (U.S. Appln. No. 08/514,866), the substance of which are incorporated herein by reference in their entirety. The performance-enhancing additives described in these documents include fluorochemical additives namely a thermally stable organic compound or oligomer containing at least one perfluorinated moiety, such as fluorochemical piperazines, stearate esters of perfluoroalcohols, and/or thermally stable organic triazine compounds or oligomers containing at least one nitrogen atom in addition to those of the triazine group. In view of their demonstrated efficacy in improving electret properties, the performance-enhancing additive is preferably a fluorochemical oxazolidinone. Preferably the fluorochemical has a melting point above the melting point of the polymer and below the extrusion temperature. For processing considerations, when using polypropylene, the fluorochemicals preferably have a melting point above 160°C and more preferably a melting point of 160°C to 290°C. Preferred fluorochemical additives include Additives A, B and C of U.S. Pat. No. 5,411,576 having the respective structures,

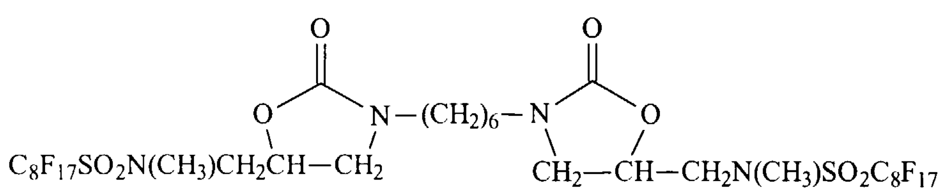

,

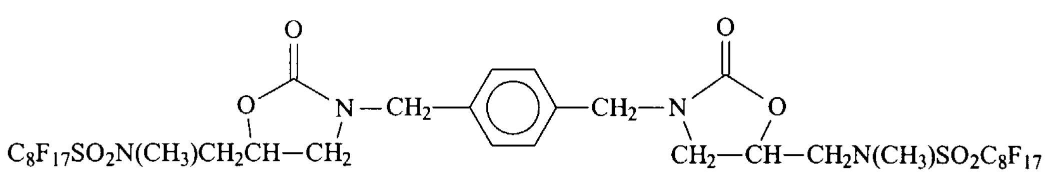

,

and

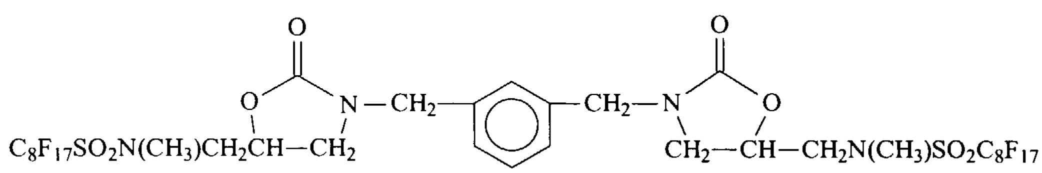

.

Preferred triazines include those having the following generic structure, where $R_2$ is an alkyl group, which

may be straight chain or branched and preferably having 4 to 10 carbon atoms and n is a number from 2 to 40, preferably 2 to 20.

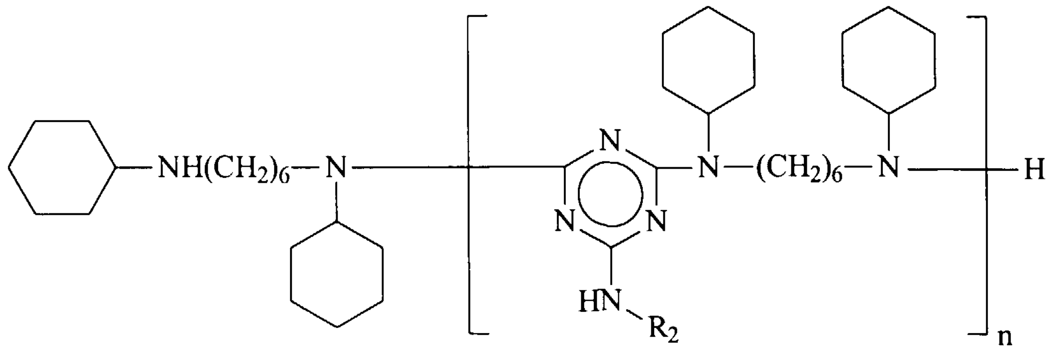

The polymer and performance-enhancing additive can be blended as solids before melting them, or melted separately and blended together as liquids. Alternatively, the additive and a portion of the polymer can be mixed as solids and melted to form a relatively additive-rich molten blend that is subsequently combined with the non-additive-containing polymer.

The performance enhancing additive is preferably a hindered or aromatic amine compound; most preferably a compound containing a hindered amine such as those derived from tetramethylpiperidine rings,

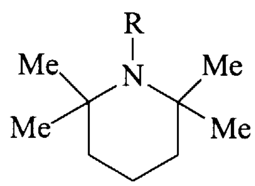

where R is hydrogen or an alkyl group. Preferably the hindered amine is associated with a triazine group as described above. Alternatively, nitrogen or metal containing hindered phenol charge enhancers could be used such as disclosed in U.S. Pat. No. 5,057,710, the substance of which is incorporated by reference in its entirety.
The nonwoven filter web of the filtration medium 1 preferably contains about 0.2 to 10 weight percent of the performance-enhancing additive; more preferably about 0.2 to 5.0 weight percent; and most preferably about 0.5 to 2.0 weight percent, based on the weight of the filter web.

In producing the melt blow fibers forming the nonwoven web of the filtration medium 1, the molten polymer or blend is preferably extruded through a fiber die onto a collecting surface. The melt polymer or blend is typically extruded from the fiber die and formed into a web of thermoplastic microfibers. The microfibers are integrally bonded each to the other at their crossover points either during the web formation process or after the web formation process. Preferably the melt polymer or blend is extruded through offices in a die under melt-blowing conditions. Melt-blowing is known to offer numerous advantages, especially at producing fine diameter nonwoven fiber webs. The melt blown webs used to form the filter webs of the filter medium 1 can be made using melt-blowing processes and apparatuses that are well known in the art. Fiber melt-blowing was initially described by Van Wente, "Superfine Thermoplastic Fibers, " Ind. Eng. Chem., vol. 48, pp. 1342-46, (1956).

In general, the melt-blowing process used to produce the filtration medium 1 is conventional, however, the conditions are modified to produce fine fiber filter webs having effective fiber diameters (EFD's) of less than 5 microns, preferably less than 4.5 microns, and most preferably less than 4 microns such as for example between 1 and 4μm. The effective fiber diameter can be decreased by decreasing the collector to die distance, using a vacuum within a foraminous collector surface, lowering the polymer flow rate, or changing the air pressure, temperature or volume used to attenuate the melt streams exiting from the die. Also, the design of the die and attenuating air vanes can be varied such as changing the relative angle of the attenuating air, changing the distance between the die tip and the junction point of the attenuating air or changing the die orifice diameters and/or orifice diameter-to-length ratios. These factors and others are discussed for example in WO 92/18677A (Bodaghi et al.). A preferred intermediate composition for making the electret filter web of the filtration medium is made by blending and extruding a mixture of 90 to 99.8 weight percent organic polymer and 0.2 to 10 weight percent of a performance-enhancing additive; wherein the material is extruded as a melt blend through a die under meltblown conditions to form fibers that are collected as a nonwoven

web. The fibers can be quenched, before being collected, by a cooling process such as water spraying, spraying with a volatile liquid, or contacting with chilled air or cryogenic gasses such as carbon dioxide or nitrogen.

Melt-blown fibers are collected as a nonwoven web on a rotating drum or moving belt. The collector to die distance is generally from 8 to 25cm, preferably from 10 to 20cm with the collector preferably being foraminous such that it can be used with a vacuum to remove excess air.

The collected web material can also be annealed to increase electrostatic charge stability in the presence of oily mists. Preferably, the annealing step is conducted at a sufficient temperature and for a sufficient time to cause the performance enhancing additive to diffuse to the interfaces (e.g., the polymer-air interface, and the boundary between crystalline and amorphous phases) of the material. Generally, higher annealing temperatures allow shorter annealing times. To obtain desirable properties for the final product, annealing of polypropylene materials should be conducted above about 100°C. Preferably, annealing is conducted from about 130 to 155°C for about 2 to 20 minutes; more preferably from about 140 to 150°C for about 2 to 10 minutes; and still more preferably about 150°C for about 4.5 minutes. Annealing should be conducted under conditions that do not substantially degrade the structure of the web. For polypropylene webs, annealing temperatures substantially above about 155°C may be undesirable because the material can be damaged.

The method for producing the filtration medium further includes the step of electrostatically charging the nonwoven web material before or after it has been collected. Examples of electrostatic charging methods useful in the invention include those described in U.S. Pat. Nos. 5,401,446 (Tsai, et al.), 4,375,718 (Wadsworth et al.), 4,588,537 (Klaase et al.), and 4,592,815 (Nakao). The electret materials may also be hydrocharged (see U.S. Pat. No.5,496,507 to Angadjivand et al.). Preferably, the charging process involves subjecting the nonwoven web to hydrocharging as disclosed in U.S. Pat. No. 5,496,507. This charging method is advantageous in that it easily provides the desired charging level in a process that can be continuous. Also this charging method can be performed on a preformed web thereby avoiding the difficulties in forming charged fibers into a uniform web structure.

Hydrocharging of the web is carried out by impinging jets of water or a stream of water droplets onto the web at a pressure sufficient to provide the web with a filtration enhancing electret charge. The pressure necessary to achieve optimum results will vary depending on the type of sprayer used, the type of polymer from which the web is formed, the type and concentration of additives to the polymer, the thickness and density of the web. Generally, pressures in the range of about 10 to 500 psi (69 to 3450 kPa) are suitable. Preferably the water used to provide the water droplets is relatively pure. Distilled or deionized water is preferable to tap water.

The jets of water or stream of water droplets can be provided by any suitable spray means. Apparatus useful for hydraulically entangling fibers are generally useful in the present invention, although operation is carried out at lower pressures in hydrocharging than generally used in hydroentangling.

The nonwoven web of filtration medium 1 has a basis weight of less than 60 grams/$m^2$, preferably from about 15 to 50 grams/$m^2$, preferably from about 20 to 40 grams/$m^2$.

The fibers can be a single layer or multiple layers or of a sheath-core configuration. If multiple layers are employed at least some of the outer layers or the sheath layer preferably contain the performance-enhancing additive as described in the blends discussed above. Preferably, the extruded fibers are in the form of microfibers having an effective diameter of less than 5 micrometers, preferably less than 4.5 as calculated according to the method set forth in Davies, C.N., "The Separation of Airborne Dust and Particulates," Inst. of Mech. Eng., London, Proceedings 1B, 1952.

Electret articles can be characterized by TSDC studies. In TSDC a sample is placed between two electrodes, heated at a constant rate, and current discharged from the sample is measured by an ammeter. TSDC is a well known technique. See, for example, U.S. Pat. No. 5,256,176, Lavergne et al., "A Review of Thermo-Stimulated Current," IEEE Electrical Insulation Magazine, vol. 9, no. 2, 5-21, 1993, and Chen et al., "Analysis of Thermally Stimulated Processes, " Pergamon Press, 1981. The current discharged from the sample is a function of the polarizability and charge trapping of the article being tested. Charged articles can be tested directly. Alternatively, charged and uncharged articles can be first poled in an electric field at an elevated temperature and then rapidly cooled below the glass transition temperature (Tg) of the polymer with the polarizing field on to "freeze in" the induced polarization. The sample is then heated at a constant rate and the resulting discharged current in measured. In the polarization process, charge injection; dipole alignment, charge redistribution or some combination of these may occur. The charged web of the filter medium is typically characterized by having a charge level as measured by TSDC of at least 5 *$Cm^2$, preferably at least 6 *C/$m^2$.

During a thermally stimulated discharge, charges stored in an electret become mobile and are neutralized either at the electrodes or in the bulk sample by recombination with charges of opposite sign. This generates an external current that shows a number of peaks when recorded as a function of temperature which is plotted on a graph (termed a TSDC spectrum). The shape and location of these peaks depends on charge trapping energy levels and physical location of trapping sites.

As indicated by many researchers (see, for example, Sessler, ed., "Electrets," Springer-Verlag, 1987, and van Turnhout, "Thermally Stimulated Discharge of Polymer Electrets," Elsevier Scientific Publishing Co., 1975), electret charges are usually stored in structural anomalies, such as impurities, defects of the monomeric units, chain irregularities et cetera. The width of a TSDC peak is influenced by the distribution of charge trapping levels in the electrets. In semicrystalline polymers, often charges will either accumulate or be depleted near the amorphous-crystalline interfaces due to the difference in conductivity of the phases (the Maxwell-Wagner effect). These trapping sites are usually associated with different trapping energies where a continuous distribution of activation energies will be expected and the TSDC peaks expected to overlap and merge into a broad peak.

The material used to form the filtration medium of the filter device of this invention is desirably substantially free of materials such as antistatic agents that could increase electrical conductivity or otherwise interfere with the ability of the web to accept and hold electrostatic charge. Additionally, the electret filtration medium should not be subjected to unnecessary treatments such as exposure to gamma rays, UV irradiation, pyrolysis, oxidation, etc., that might increase electrical conductivity. Thus, in a preferred embodiment the filtration medium is made and used without being exposed to gamma irradiation or other ionizing irradiation.

A high efficiency filtration medium for the filter device preferably comprises a filter web of electret charged fibers of a nonconductive thermoplastic resin such as for example polypropylene, poly(4-methyl-1-pentene) or blends thereof which have a basis weight (BW) of less than 60 grams/m$^2$, an effective fiber diameter (EFD) of less than 5 microns and a penetration (PEN) of less than 0.03%, wherein the ratio (I);

$$BW/(EFD*PEN) \qquad I$$

is greater than 200, preferably greater than 400, most preferably greater than 1,000. The high efficiency filter web percent penetration (PEN) is preferably less than 0.02%, and most preferably less than 0.01%. Percent penetration is defined as particle concentration downstream versus particle concentration upstream for 0.3 micron sodium chloride particles at a face velocity of 5.3 cm/sec.

The filtration medium basis weight is preferably less than 50 grams/m$^2$, and more preferably 40 grams/m$^2$ or less. The thickness of the filter web is generally less than 0.15 cm, preferably less than 0.1 cm.

The filtration medium of the filter device of the invention can be easily corrugated into conventional zig-zag pleats with high pleat density due to its low basis weight and thickness. The filtration medium can be corrugated into pleated structures by standard pleating methods and equipment. This pleatability and handleablity is due to the relatively high strength of the melt formed thermoplastic fiber webs. Preferably the nonwoven filter webs have a tensile strength sufficient to be self-supporting, which generally is a tensile strength in at least one direction of at least about 5 Newtons, preferable at least 10 Newtons, and is formed of a matrix of entangled microfibers that are bonded together at the fiber crossover points.

The filtration medium web can be used alone or it can be laminated to further functional layers by adhesives, heat bonding, ultrasonics, co-pleating or the like. These further functional layers can be prefilter layers, e.g. for large diameter particles; support layers such as scrims, spunbond, spun lace, melt blown, air laid nonwoven, wet laid nonwoven; absorbent filtration medium; protective cover layers or any other functional layer or layers. Also multiple layers of filter web can be laminated together to provide a filter medium of the filter device with improved performance. For example, webs prepared in accordance with the above described method which may have insufficient performance so as to obtain a filter device with the required efficiency can be laminated together to obtain the required efficiency of the filter device. These laminated webs can exhibit higher efficiencies than those obtainable for comparable singe layer webs of the same basis weight.

In accordance with a particular embodiment of the present invention, the filter medium 1 is laminated to a layer comprising a hygroscopic material providing heat and moisture exchange capability to the filter device. For example, a non-woven web of superabsorbent fibers as disclosed in US 5,577,494 can be laminated to the filter medium of the filter device. Alternatively, a nonwoven layer of hydrophilic fibers impregnated with a hygroscopic substance as disclosed in US 4,771,770 may be laminated to the filter medium of the filter device. The pleated filter medium of the filter device may further be combined with a heat and moisture exchange material as disclosed in US 5.320.096.

Because of the high efficiency that can be achieved with the filter medium used in the filter device of the invention, it is furthermore possible to combine without laminating one to the other, a separate heat and moisture exchange filter with the pleated filter medium for capturing bacteria and virus material in the fluid chamber of the filter device without having to increase the volume of the filter device. For example, the filter device may comprise in the fluid chamber the pleated filter medium and a heat and moisture exchange filter in the form of a flat bobbin as disclosed in for example WO 94/02192.

On the other hand, if a heat and moisture exchange filter is not needed, the use of the high efficiency filter medium provides the possibility of decreasing the volume of the fluid chamber as the volume occupied by the filter medium of the filter device of the invention is substantially less than that occupied by a glass fiber based filter. For example, it is possible without sacrificing the desired efficiency to reduce the volume of the fluid chamber to less than 100ml and often to less than 60 ml or even less than 40 ml. This provides the advantage that the volume of air residing

in the fluid chamber and which therefore does not get frequently refreshed can be further reduced.

The invention is further illustrated by way of the following examples without the intention however to limit the invention thereto.

EXAMPLES

Preparation of the filter

The following polypropylene resin composition was prepared:

CHIMASSORB™ 944FL (a hindered amine available from Ciba-Geigy Corp., Hawthorne, NY) was melt compounded into poly(4-methyl-1-pentene) (TPX DX 820, available from Mitsui Petrochemical Industries, Tokyo, Japan) in a single screw extruder in a 40:60 ratio and the resultant blend was extruded into a large diameter fiber. The fiber was subsequently ground into a powder (0.125 inch mesh) which was added to the polypropylene pellet feed during preparation of the BMF webs. The polypropylene resin composition consisted of 98 wt.% polypropylene, 1.2 wt.% poly(4-methyl-1-pentene), and 0.8 wt.% CHIMASSORB™ 944FL

A polypropylene based blown microfiber (BMF) web was prepared using a melt blowing process similar to that described, for example, in Wente, "Superfine Thermoplastic Fibers," in Industrial Engineering Chemistry, Vol. 48, pages 1342 et seq (1956) or in Report No. 4364 of the Naval Research Laboratories, published May 25, 1954, entitled "Manufacture of Superfine Organic Fibers" by Wente et al.. The extruder had four temperature control zones which were maintained at 210°C, 290°C, 340°C, and 340°C, the flow tube connecting the extruder to the die (J&M MPMB Die (Melted Polymer Melt Blown Die) available from J&M company (Dawsonville, GA) ) was maintained at 340°C, and die was maintained at 340°C. The primary air was maintained at about 400°C and 690 kilopascals (kPa) with a 0.102 cm gap width, to produce a uniform web. The polypropylene resin composition described above was delivered from the die at a rate of 0.16 g/hole/min. and the resulting web collected on a perforated rotating belt collector. The collector table was connected to a vacuum system which could be optionally turned on or off while collecting the BMF web, thereby allowing a higher solidity web to be prepared when a vacuum was applied to the collector table. Cold air plenums were positioned intermediate of the die and the collector table to facilitate cooling the BMF web and increasing overall production rates. In all cases, lower basis weight BMF webs were obtained by increasing the rotational speed of the collector rather than reducing the resin delivery rate. The average effective fiber diameter (EFD) for the webs produced was 2.7μm. The web basis weight data is indicated for each example below.

The webs produced were charged using a hydro-charging process substantially as described in U.S. Patent No. 5,496,507 (Angadjivand et al.) using a water pressure of about 550 kPa.

Preparation of the filter devices

The filter web prepared as described above was provided on both sides with a cover web of 15g/m$^2$. This construction was further provided on both sides with a netting (Vexar® L 190 available from Dupont, Canada) to yield the filter medium.

Three filters designated A, B and C were produced by inserting the filter medium into a cylindrical fluid chamber and sealing the filter medium to the fluid chamber walls using the so-called spin coating method. In this method, a polyurethane resin (sealant) is dropped into the filter device while rotating that device. This forces the polyurethane resin outwardly to the interface of the filter medium and the fluid chamber wall. This procedure was continued until the polyurethane resin started to harden. The filter devices A, B and C differed in their configuration as indicated in the following table:

| | A | B | C |
|---|---|---|---|
| Approximate filter diameter | 50 mm | 70 mm | 70 mm |
| Pleat height | 12 mm | 15 mm | 15 mm |
| Distance between adjacent pleats | 2-4 mm | 2-4 mm | 2-4 mm |
| Basis weight of the web | 20 g/sqm | 40 g/sqm | 20 g/sqm |
| Volume of the fluid chamber | 55 ml | 99 ml | 99 ml |

Comparative filter device

For comparison, a commercially available glass fiber based filter PALL™ BB 100 available from Pall Corporation USA was used. This filter device has a box shaped fluid chamber with a volume of 100 ml. The filter web of the comparative filter is pleated with a pleat height of 25 mm and a pleat distance of 2.15mm. This filter device is hereinafter designated with the letter D.

Test methods

1. Sodium chloride filter efficiency

The efficiency of the filters was measured using the apparatus AFT8130 from TSI Inc., and according to U. S. Standard 42 CFR 84 which uses sodium-chloride crystals generated from a solution which is sprayed so that water as the solvent is evaporated resulting in sodium-chloride crystals with a typical size of 0.3 microns. The flow rate used was 30 l/min.

2. Bacterial (BFE) and virus (VFE) removal efficiency

The ability of the filter devices to remove micro-organisms is measured by nebulizing viable micro-organisms into an airstream and passing them through the filter device. The test micro-organisms used provide both a bacterial spore (Bacillus subtilis) and a viral (MS-2 coliphage) challenge to the filter device. The performance of the filter device is assessed by measuring the number of viable micro-organisms in the airstream after the airstream has passed through the filter device and comparing this with the number of viable micro-organisms in the challenge (i.e. in the airstream as it reaches the filter device). The performance is measured as a percentage of micro-organisms that were removed from the airstream by the filter device. The flow rate used was 30 l/min.

3. Hydrostatic pressure

Hydrostatic pressure was measured according to the AATCC 127-1995 test method.

4. Pressure drop

Pressure drop was measured while measuring efficiency of the filter using the AFT8130 form TSI Inc.. The pressure drop was measured a flow rate of 30 l/min.

The following table summarizes the results obtained for the three invention filter devices (A,B,C) and the comparative filter device (D).

| Filter | NaCl test (%) | BFE (%) | VFE(%) | HP[1] | PD[2] |
|---|---|---|---|---|---|
| A | 99.995 | 99,9998360 | 99,9986000 | 100 | 11,26 |
| B | 99.999 | 99,9999930 | 99,9999999 | 100 | 9,22 |
| C | 99.997 | 99,9996560 | 99,9996770 | 100 | 6,88 |
| D | 99.990 | 99,9999800 | 99,9986000 | 93 | 11,60 |

[1] Hydrostatic pressure measured in cm of water
[2] Pressure drop measured in mm of water

From the above table it can be seen that the filter devices of the invention have an improved hydrostatic pressure and lower pressure drop. All filter devices of this invention have the same or even improved VFE performance as the comparative filter and have similar performance with respect to BFE performance although filters A and C have a slightly lower BFE than the comparative filter device.

**Claims**

1. A filter device for connection to the respiratory tract of a patient, comprising a housing having a fluid chamber connecting to two fluid ports, the fluid chamber having a volume of not more than 140 ml and comprising a pleated filtration medium for capturing bacteria and viral material characterised in that the pleated filtration medium comprises a nonwoven web of electret charged fibers of a nonconductive thermoplastic resin, having a resistivity of at least $10^{14}$ ohm-cm, said electret charged fibers having an effective diameter (EFD) of not more than 5 µm and

said filter device having an efficiency of at least 99.97% for sodium chloride particles of 0.3µm at a flow challenge of 30 l/min.

**2.** A filter device according to claim 1 wherein the basis weight (BW) of said nonwoven filter web is less than $60g/m^2$.

**3.** A filter device according to claim 2 wherein the basis weight (BW) of said nonwoven web is between $10g/m^2$ and $40g/m^2$.

**4.** A filter device according to claim 2 or 3 wherein the penetration (PEN) of the nonwoven filter web is less than 0.03% and wherein the ratio BW/(EFD*PEN) is more than 200.

**5.** A filter device according to any of the previous claims wherein the thermoplastic resin is a polyolefin.

**6.** A filter device according to any of the previous claims wherein the effective diameter is between 1 and 4µm.

**7.** A filter device according to any of the previous claims further comprising a heat and moisture exchange filtration medium.

**8.** A filter device according to claim 7 wherein said heat and moisture exchange filtration medium is a substrate impregnated with a hygroscopic material.

**9.** A filter device according to any of the previous claims wherein said fluid chamber comprises a cylindrical portion.

**10.** A filter device according to any of the previous claims wherein said fluid chamber comprises a central portion and wherein said fluid ports have a diameter less than the cross-section of said central portion.

**11.** A filter device according to any of the previous claims wherein one of said fluid ports is connected to a conduit that is in fluid communication with the trachea of a patient and wherein the other fluid port is connected to a further conduit connecting to a breathing apparatus.

**12.** A filter device according to any of the previous claims wherein the volume occupied by said filtration medium is less than 60ml.

**13.** Method of filtering breathable air comprising the steps of passing breathable air to be inspired by a patient through the filter device of any of claims 1 to 12 and passing air expired by the patient through the filter device of any of claims 1 to 12.

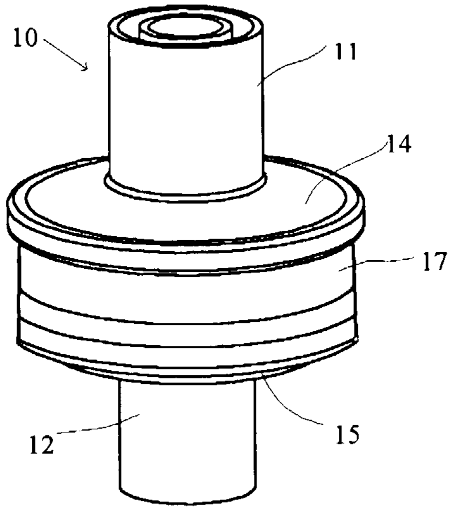


Figure 1

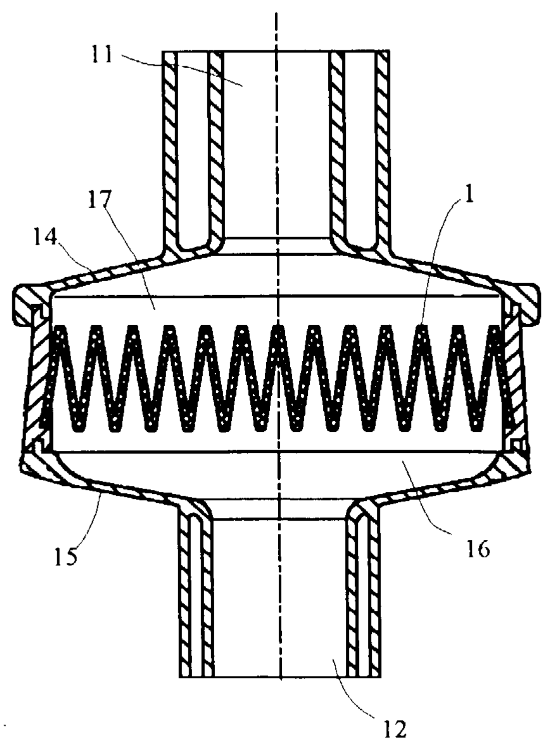


Figure 2

European Patent Office

# EUROPEAN SEARCH REPORT

Application Number
EP 99 20 2349

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 0 845 554 A (MINNESOTA MINING & MFG) 3 June 1998 (1998-06-03) * page 2, line 44 - line 51 * * page 3, line 3 - line 14 * * page 3, line 22 - line 27 * DOP Penetration and Pressure Drop * page 4, line 41 - line 57; example CII * --- | 1-6 | B01D39/16 A61M16/10 |
| A | US 5 240 479 A (BACHINSKI THOMAS J) 31 August 1993 (1993-08-31) * abstract * * column 5, paragraph 3 * * column 6, line 13 - column 7, paragraph 3 * * column 13, line 10 - line 45; figure 3 * ----- | 1,4,5,9, 10 | |

TECHNICAL FIELDS SEARCHED (Int.Cl.7)
B01D
A61M
A62B

The present search report has been drawn up for all claims

| Place of search | Date of completion of the search | Examiner |
|---|---|---|
| THE HAGUE | 29 December 1999 | Plaka, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 20 2349

This annex lists the patent family members relating to the patent documents cited in the above-mentioned European search report.
The members are as contained in the European Patent Office EDP file on
The European Patent Office is in no way liable for these particulars which are merely given for the purpose of information.

29-12-1999

| Patent document cited in search report | Publication date | Patent family member(s) | Publication date |
|---|---|---|---|
| EP 0845554 A | 03-06-1998 | AU 680561 B | 31-07-1997 |
| | | AU 7953294 A | 14-03-1995 |
| | | BR 9407259 A | 24-09-1996 |
| | | CA 2168126 A | 23-02-1995 |
| | | CN 1129963 A | 28-08-1996 |
| | | DE 69417041 D | 15-04-1999 |
| | | DE 69417041 T | 15-07-1999 |
| | | EP 0714463 A | 05-06-1996 |
| | | ES 2128590 T | 16-05-1999 |
| | | JP 9501604 T | 18-02-1997 |
| | | PL 312993 A | 27-05-1996 |
| | | WO 9505501 A | 23-02-1995 |
| | | US 5496507 A | 05-03-1996 |
| US 5240479 A | 31-08-1993 | NONE | |

EPO FORM P0459

For more details about this annex : see Official Journal of the European Patent Office, No. 12/82